# EUROPEAN PATENT SPECIFICATION

(11) **EP 4 555 452 B1**
(45) Date of publication and mention of the grant of the patent: **11.11.2026**
(21) Application number: 23747997.7
(22) Date of filing: 06.07.2023
(51) Int. Cl.: G06N 3/0895, G06N 3/096, G16H 30/40, G16H 50/70

(54) **IMAGE ANALYSIS SYSTEMS AND METHODS UTILIZING NEURAL NETWORKS ADAPTED FOR CONTINUOUS LEARNING**
VERFAHREN ZUM INKREMENTELLEN TRAINIEREN EINES NEURONALEN NETZWERKS UNTER VERWENDUNG VON BILDAUGMENTATION MITTELS MOBIUS-TRANSFORMATION
MÉTHODE DE FORMATION INCRÉMENTIELLE D'UN RÉSEAU DE NEURONES UTILISANT L'AUGMENTATION D'IMAGE AVEC LA TRANSFORMATION DE MOBIUS

(30) Priority: 11.07.2022 EP 22184037
(43) Date of publication of application: 21.05.2025
(73) Proprietor: UCB Biopharma SRL, 1070 Brussels (BE)
(72) Inventor: PATRA, Arijit, Brussels (BE)
(74) Representative: J A Kemp LLP
(86) International application number: PCT/EP2023/068743
(87) International publication number: WO 2024/012988

(56) References cited:
- ZHU FEI ET AL: "Prototype Augmentation and Self-Supervision for Incremental Learning", 2021 IEEE/CVF CONFERENCE ON COMPUTER VISION AND PATTERN RECOGNITION (CVPR), IEEE, 20 June 2021 (2021-06-20), pages 5867 - 5876, XP034008626, DOI: 10.1109/CVPR46437.2021.00581
- SHARON ZHOU ET AL: "Data augmentation with Mobius transformations", ARXIV.ORG, CORNELL UNIVERSITY LIBRARY, 201 OLIN LIBRARY CORNELL UNIVERSITY ITHACA, NY 14853, 7 June 2020 (2020-06-07), XP081682313
- CASTRO FRANCISCO M ET AL: "End-to-End Incremental Learning", 6 October 2018, PATTERN RECOGNITION : 5TH ASIAN CONFERENCE, ACPR 2019, AUCKLAND, NEW ZEALAND, NOVEMBER 26-29, 2019, REVISED SELECTED PAPERS, PART II; [LECTURE NOTES IN COMPUTER SCIENCE], SPRINGER INTERNATIONAL PUBLISHING, CHAM, PAGE(S) 241 - 257, ISBN: 978-3-030-41298-2, ISSN: 0302-9743, XP047635193

## Description

The present invention relates to systems and methods of training and generating deep neural networks over temporally spaced arrivals of unrelated information, using augmentation based on fractional linear transformations as a part of the process.

### BACKGROUND

Deep learning-based methods have become common in medical imaging research. In realistic situations, clinical imaging systems often do not have access to all the re-quired data initially, but data arrives in incremental chunks over time, acquired with multiple devices and across different centers. This problem is pronounced in healthcare systems in low and middle income countries (LMIC) where data acquisition and quality assurance infrastructure may not be as developed [Becker et al, Tropical Medicine & International Health 21.3 (2016), pp. 294-311]. Such cases of variable data accessibility require machine learning algorithms to be robust to adaptations on new data distributions over time and be generalizable to novel classes of data, in order to remain clinically significant and reliably aid diagnostic efforts throughout their shelf lives un-der evolving requirements. This requirement for continual adaptation in deep networks for clinical imaging implies a need to ensure that model parameters remain relevant to both old and new tasks in incremental data regimes. This needs to occur without storing large numbers of exemplars from past classes over subsequent learning schedules owing to constraints on long-term storage of clinical data in terms of memory, legal and privacy issues [see e.g. FDA, et al. Proposed regulatory framework for modifications to artificial intelligence/machine learning (AFML)-based software as a medical device (SaMD)-discussion paper. 2019]. Thus, the ideal joint training condition of optimizing models with all datasets ever used at each incremental retraining is challenging in clinical imaging.

Adaptation of existing models to learn new classes was attempted by transfer learning [Ravishankar, et al Deep Learning and Data Labeling for Medical Applications, pp. 188-196 (2016)]. Transfer learning, despite helping prior learning episodes to enhance future task learning, was found to inefficiently balance old and new task knowledge in the ultimately available models. Studies show a decline in past performances or catastrophic forgetting [Goodfellow et al. arXiv:1312.6211 (2013)], as information previously learnt is lost causing high validation losses on past data. Recent work has pursued mitigation of forgetting in deep networks with parameter expansion [Rusu et al arXiv:1606.04671 (2016)], exemplar replay [Li et al IEEE Transactions on Pattern Analysis and Machine Intelligence 40(12), pp. 2935-2947 (2017)], generative rehearsal [Kemker et al. arXiv:1711.10563 (2017)] and weight regularization [Kirkpatrick et al Proceedings of the national academy of sciences, pp. 3521-3526 (2017)]. Knowledge distillation, where representations learnt by a model are transferred to another, are often used in model compression [Hinton et al NIPS 2014 Deep Learning Workshop (2014)]. It has been used for incremental learning as the representation from one learning session can help regularize a future session, with the old tasks' logits regularizing the learning on new data. Such methods include Learning without Forgetting (LwF) with distillation and cross-entropy objectives, iCaRL which incrementally learns representations, Learning without Memorizing (LwM) where distillation and class activation run continually, progressive retrospection (PDR) using distillation from both old and new models.

In clinical imaging, data availability is often not immediate and models learning incrementally over time without affecting past performance have been researched, such as pixel regularization for MRI segmentation, modelling Alzheimers progression, weight consolidation and distillation, hierarchical continual learning etc. While data augmentation has been extensively used in machine learning, there has been relatively little research on runtime augmentation on examples retained in incremental learning.

Augmentation approaches have been used in training of deep neural networks as a regular process. The same is true for Mobius augmentation as originally demonstrated in [S. Zhou et al, "Data augmentation with Mobius transformations", arXiv:2002.02917 (2020)], which discloses a method in which Möbius transformations are applied to input images for data augmentation with the aim of improving generalization in low data regimes. The utilization of such augmentation methods have been challenging and inadequate in related literature, including [F. M. Castro et al, "End-to-End Incremental Learning", Computer Vision - ECCV 2018, Lecture Notes in Computer Science, vol. 11216, pages 241-257 (2018)], which discloses a method that retains exemplar training images from old classes and applies data augmentation both to the retained exemplars and samples from new classes with the aim of reducing catastrophic forgetting. The adoption of augmentation methods in incremental and continual learning, as opposed to regular deep neural network training, is technically challenging, particularly in the regime of limited data availability (a scenario specifically targeted by the present disclosure, and not considered by the prior art). The inclusion of a fractional linear transformation in the training process when datasets arrive over incremental time intervals has been until now an unexplored approach, as it is a non-trivial inclusion in the light of the catastrophic forgetting phenomenon highlighted in the prior art. Prior art in the field relies on simple incremental distillation techniques or geometric transformations for stored exemplars, rather than a mathematical transformation of the input space.

### SUMMARY OF THE INVENTION

The present disclosure relates to designs of neural networks adapted for learning over temporally spaced arrivals of relevant data, in a process known as 'Continual Learning' or 'Lifelong Learning' or 'Incremental Leaming'. The disclosure provides systems and methods of training and generalizing deep neural networks over temporally spaced arrivals of unrelated information, using augmentation based on fractional linear transformations as a part of the process.

The present invention provides a novel approach for incremental training of a neural network without storing a large number of samples in the analysis of images. This approach is exemplified using a dataset of colorectal carcinoma images to show a proof-of-concept. This is achieved by propagating sample diversity through a novel online augmentation over a limited number of past tasks' samples, while performing a weighted cross-distillation over the logits of the past classes while training on new class data for the available model. The key differences from existing approaches are: a) a concept of incremental time data augmentation strategy using Mobius transformations b) weighted cross-distillation for continual learning of new classes c) an online adaptation of Mobius augmentation in incremental learning tasks.

The present disclosure provides a method of incremental learning relying on Mobius transformations and its interpretation as a composition of elementary operations like translation, rotation and so on, which individually form the basis of many sample-level data augmentation methods. The method further uses distillation approach where class specific accuracy is used to apportion importance to the over-all past logits vector. The combination of the two steps involves a few samples from old classes being subjected to an online augmentation using Mobius transformation to improve representation of previously seen classes as the model is optimized for new classes. During the optimization over new classes, the old class logits after being weighted and summed up to reflect an overall representation of old tasks allow the model to have a snapshot of the past learning and prevent a catastrophic perturbation to the parameter space along with cross-entropy optimization that enables the new task learning to account for both old class knowledge and the new class sample information. The invention is set out in the appended set of claims.

### BRIEF DESCRIPTION OF THE DRAWINGS

The present invention is described below by reference to the following drawings, in which:

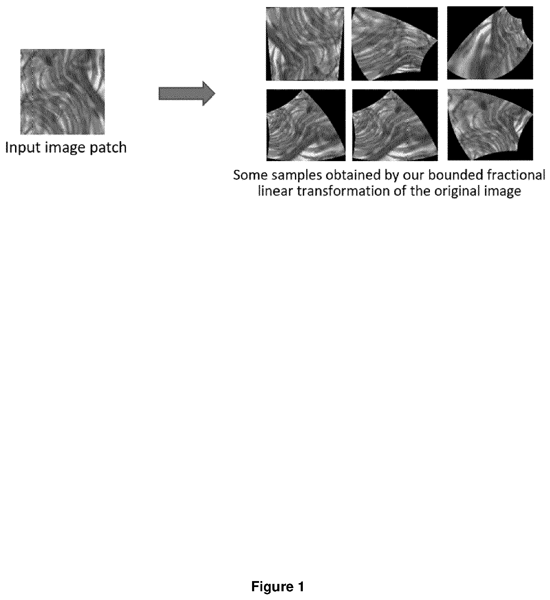

Figure 1 shows sample images from the colorectal histology dataset used (top); Mobius transformed augmented examples during incremental learning (bottom)

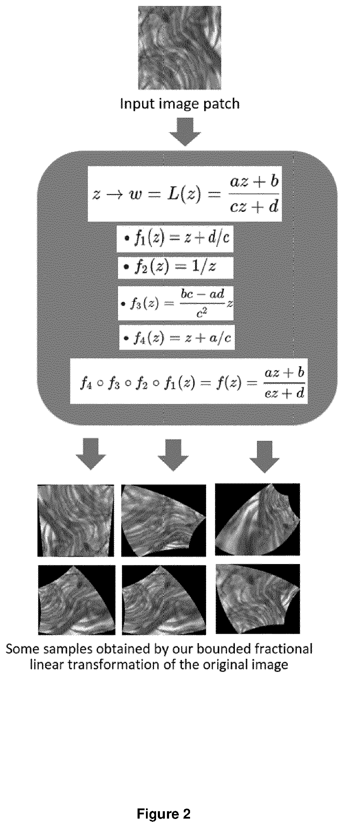

Figure 2 demonstrates interpretation of Mobius transformations as a composition of basic transformations enables an algorithmic implementation to plug into the incremental learning step at real-time.

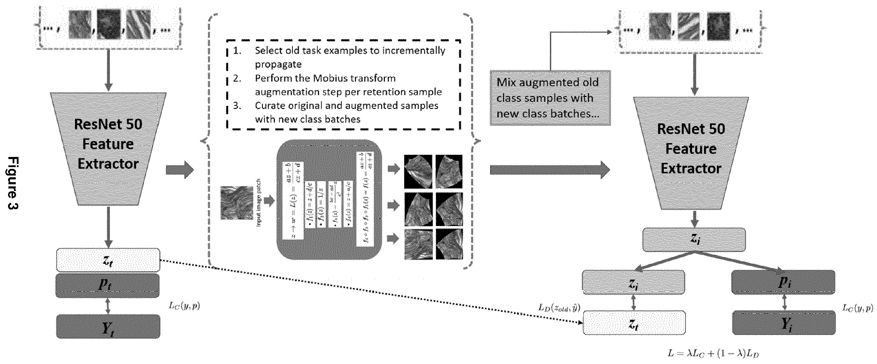

Figure 3 is an Illustration of our overall pipeline. The initial training is performed (left) followed by a curation of old task exemplars, a Mobius augmentation step and interspersing with new class batches, followed by incremental task training under joint cross-entropy and distillation (right).
Figure 4 shows accuracy (in %) for Task 2/Stage 2 classes. Benefits of forward transfer on new class data is evident (left); *ΔAcc* comparison of Mobius transforms and existing methods for augmentation on select old task exemplars over distillation and finetuning (right).

### DETAILED DESCRIPTION OF THE INVENTION

### Problem addressed

Prior art, related to continual/incremental learning, does not indicate the integration of mathematical transformations of an input data as a basis for effective knowledge transfer under the conditions of limited data availability during incremental learning (in itself, this training methodology and the allied systems/methods are distinct from the standard process of machine learning in the aspects that the latter process of learning from data is a static process which does not account for dynamic, temporally-spaced arrivals of novel information, which continual/incremental learning seeks to achieve).

The technical problem addressed by the present disclosure is achieving viable incremental learning performances in situations of limited data availability in incremental learning settings, particularly for clinical/preclinical histology data, is solved by the different methodological approaches proposed, specifically the usage of Mobius transformations on the input space. The usage of distillation processes themselves are known from the prior art, but such processes alone do not allow for effective generalization to incremental regimes when imaging data (such as histology data) is considered under limited data availability. Such an ability is gained, as shown by the present disclosure, by an integration of mathematical transformations of the input space in incremental time. The disclosure provides for the first time the usage of such transformations in an incremental learning process and demonstrates that the resultant effects on the input space of datasets.

The overall pipeline of continual learning provided by the present disclosure addresses a two-fold technical problem of learning dynamically over temporally spaced arrivals of datasets and of enabling such incremental learning over relative few exemplars (few-shot learning) representative of such a dynamic arrival of data classes in clinical/preclinical histology contexts. The usage of mathematical transformations on a data input space, such as Mobius transformations, has only been explored in prior art as data augmentation strategies in static machine learning methods. The disclosure provides methods that integrate such transformations in a dynamic incremental learning context, as a sub-part of the overall distillation-driven continual learning procedure. The present disclosure provides an approach not discussed or suggested by the prior art by enabling such continual learning procedures for limited data availability, or in situations representative of the complexities of dealing with histology datasets.

Consider a problem where the model needs to be trained in an *M*-stage fashion, with each stage being a classification task with classes as *Xt = {Xt,i}^{Kt}i=1, t ∈ [1,M],* with each *X* being a class and includes samples *xt ∈ Xt* and *Kt* being the number of classes in each stage *t.* The class ifier learning in stage *t-1,* after incrementally being optimized over the classes at the *t^{th}* stage, shouldn't show marked de-clines in inference capacity over validation set instances from *(t-1)^{th}* stage or prior stages. Here, we design an incremental learning experiment with four classes in the initial training stage and four in the incremental stage *(M=2, KI=K2=4).*

This study is modelled as a sequential class learning task as above, with a proportion of classes being learnt as 'base classes' during an initial training stage. Next, the remaining classes are learnt as 'incremental classes' in a subsequent learning stage, leading to a multistage learning system over a temporal interval. The former are used to optimize for the initial task (Task 1) and the latter help train the model trained over base classes for the incremental task (Task 2), thus simulating a continual learning scenario.

### Mobius augmentation

Many sample-level data augmentation methods at training time belong to a set of affine transformations, which includes a group of mappings like rotation, scaling, translation and flipping. Such operations can be modelled as a bijective mapping in a complex plane as z → *az + b,* where the variable z, parameters *a, b ∈ C,* the set of complex numbers.

A generalization of this mapping considers the presence of non-zero imaginary parts of the complex numbers in the transformation and the affine mapping being performed in the Argand plane [Özdemir et al. Communications in Nonlinear Science and Numerical Simulation, 16(12): 4698-4703, 2011]. This expands the superset of possible image transformations with valid label preservation. The denominator of a linear transformation z → *az +* b can be assumed as unity. This can also be obtained by treating the denominator as a complex number cz *+ d,* such that the real part of this complex quantity is unity and the imaginary part is zero. This hints at the next stage of abstraction by introducing a denominator with non-zero real and imaginary components *(c, z≠* 0). This creates a group of transformations in the set of complex numbers: $f \left(z\right) = \left(az+b\right) / \left(cz+d\right)$ (1) where *a,b,c,d ∈ C* and *ad - bc ≠* 0 is the invertibility condition.

This encapsulates a superset of basic mappings including inversion, translation, rotation and flipping and is termed a Mobius transformation if z *∈ C, f(z)* is not constant and *cz+d≠ 0* [19]. A point z is mapped from one complex plane to another using parameters *a,b,c,d.* This can proceed without an explicit imaginary part defined for the complex entity z, as every real number can have a form x + *iy,* where *x ∈ R,* and *y* = *0.* This enables us to define points on the image to estimate *a, b, c* and d. We choose 3 points at random on the image space with different combinations allowing for a different output at the conclusion of the mapping operation with label information preserved. This allows expansion in sample diversity per input in available datasets, with a much larger set of possible modifications for a particular class compared to existing sample-level methods. With a transformed appearance in 2D, the Mobius augmentation improves model generalization and robust-ness to noise and dataset shifts. Assuming 3 points in the initial plane as *z1, z2,* z3 and in a target plane as *w1,* w2, w3, then considering the preservation of anharmonic ratios [19]: $\frac{\left(w-{w}_{1}\right) \left({w}_{2}-{w}_{3}\right)}{\left(w-{w}_{3}\right) \left({w}_{2}-{w}_{1}\right)} = \frac{\left(z-{z}_{1}\right) \left({z}_{2}-{z}_{3}\right)}{\left(z-{z}_{3}\right) \left({z}_{2}-{z}_{1}\right)}$ (2) $\frac{\left(w-{w}_{1}\right)}{\left(w-{w}_{3}\right)} = \frac{\left(z-{z}_{1}\right) \left({z}_{2}-{z}_{3}\right) \left({w}_{2}-{w}_{1}\right)}{\left(z-{z}_{3}\right) \left({z}_{2}-{z}_{1}\right) \left({w}_{2}-{w}_{3}\right)}$ (3) where, $w = \frac{\left(A{w}_{3}-{w}_{1}\right)}{A - 1}$ $A = \frac{\left(z-{z}_{1}\right) \left({z}_{2}-{z}_{3}\right) \left({w}_{2}-{w}_{1}\right)}{\left(z-{z}_{3}\right) \left({z}_{2}-{z}_{1}\right) \left({w}_{2}-{w}_{3}\right)}$

The transformation function in a reduced form can be expressed as: $f \left(z\right) = w = \frac{\left(A{w}_{3}-{w}_{1}\right)}{A - 1} = \frac{az + b}{cz + d}$ (4)

Subsequently, the values of coefficients *a,b,c,d* in terms of the chosen points *(z1, z2, z3)* and *(w1, w2, w3)* can be obtained through substitution in equations (1), (3) and (4): $a = {w}_{1} {w}_{2} {z}_{1} - {w}_{1} {w}_{3} {z}_{1} - {w}_{1} {w}_{2} {z}_{2} + {w}_{2} {w}_{3} {z}_{2} + {w}_{1} {w}_{3} {z}_{3} - {w}_{2} {w}_{3} {z}_{3}$ (5a) $b = {w}_{1} {w}_{3} {z}_{1} {z}_{2} - {w}_{2} {w}_{3} {z}_{1} {z}_{2} - {w}_{1} {w}_{2} {z}_{1} {z}_{3} + {w}_{2} {w}_{3} {z}_{1} {z}_{3} + {w}_{1} {w}_{2} {z}_{2} {z}_{3} - {w}_{1} {w}_{3} {z}_{2} {z}_{3}$ (5b) $c = {w}_{2} {z}_{1} - {w}_{3} {z}_{1} - {w}_{1} {z}_{2} + {w}_{3} {z}_{2} + {w}_{1} {z}_{3} - {w}_{2} {z}_{3}$ (5c) $d = {w}_{1} {z}_{1} {z}_{2} - {w}_{2} {z}_{1} {z}_{2} - {w}_{1} {z}_{1} {z}_{3} + {w}_{3} {z}_{1} {z}_{3} + {w}_{2} {z}_{2} {z}_{3} - {w}_{3} {z}_{2} {z}_{3}$ (5d)

Based on Liouville's theorem [Liouville, J., Extension au cas des trois dimensions de la question du tracé géographique. Note VI, pages 609-617, 1850], a Mobius transformation can be expressed as a composition of translations, orthogonal transformations and inversions, encompassing a superset of a number of common augmentation operations in deep learning. This helps us design an algorithmic framework for real-time generation of Mobius transformations using values *of a, b, c, d* from (5a,5b,5c,5d) to form subspaces of compositions on basic transformations from a superset of the generalized Mobius transformation. While an infinite number of Mobius samples can be obtained, the number of samples is bounded by randomly assigned cutoffs at runtime within [1, R], where R is the maximum number of samples allowed by memory constraints. In the exemplary implementation below R was set at 250 based on available RAM settings in the exemplary implementation.

### Weighted distillation

Representations learnt by models can also be thought of as representing a 'dark knowledge' [Hinton et al NIPS 2014 Deep Learning Workshop (2014)] about the model-data dynamics in a compact vectorized form. This process was termed as knowledge distillation since the heavier models' learning is 'distilled' into an essential, compact representation that can be used in the other tasks. The current disclosure method uses vector as a 'memory' of past class learning to regularize incremental training. Based on the initial learning, class averaged logits are retained per class by saving to memory the validation logits at the conclusion of the training sched-ule of the initial (Task 1) training. Next, the weighted logits are computed by applying weighting factors to logits of individual classes, the weights being numerical inverses of class-specific validation accuracies. This allows the distillation logits to reflect class-wise biases in proportion to their difficulty for the model to learn. The initial classes' training employs a cross-entropy loss. The probability vector of the initial task, is *p = softmax (z)* ∈ 1 , where z is the set of logits. The objective in the initial training stage: ${L}_{crossent} \left(y, p\right) = - {\sum }_{i = 1}^{{K}_{1}} {y}_{i} . log \left({p}_{i}\right)$ (6)

Here *pi* is the predicted probability score vector for each class in the new task, *yi* is the associated ground truth in a one-hot encoding form. In next sessions, a distillation term is added to the objective, to enable representation of past knowledge in the learn-ing process (*y*' are final layer class scores for new task classes before softmax steps): ${L}_{distillation} \left({z}_{old},y′\right) = - {\sum }_{i = 1}^{N} softmax \left(\frac{{z}_{old}}{T}\right) . log \left(softmax\left(\frac{y {′}_{i}}{T}\right)\right)$ (7)

Logits and predictions are scaled with a temperature term T in a softening process. Softening with a temperature hyperparameter helps reduce the disparity between the class label with the highest confidence score in the probability vector with respect to the other class labels and helps better reflect inter-class relationships at the representation learning stage. Considering the overall logit vector for old classes, after weighting as *zold,* class-specific logits are weighted to obtain a sum of class-weighted logits as: ${z}_{old} = {\sum }_{i = 1}^{{K}_{1}} {u}_{i} . {z}_{i}$ (8)

The logits from individual classes *zi, i ∈ [1,K1]* are calculated by averaging pre-soft-max probability values (after sigmoid activation) for examples from each of KI classes. The weights *(ul,u2, ...,uk1* ) are computed as inverse of class-specific accuracy on validation sets of the initial classes. The idea is to boost logits from classes which are inherently difficult to learn for the model (lower the class-specific accuracy, higher the class weight). This reduces the disparity among classes in their contribution towards the overall sessional representation vector to be saved as an imprint of Stage 1 learning. Overall, the net incremental objective for learning beyond initial sessions is (y = 0.5): $L = γ {L}_{crossent} + \left(1-γ\right) {L}_{distillation}$ (9)

### Method for continuous training a neural network

The present disclosure provides a novel method of continual machine learning using Mobius transformations for online augmentation. Subsequently, the disclosure demonstrates the value of the generalized Mobius transformations for performing augmentation of an exemplar set in a distillation-based incremental learning setting, introducing a new concept of incremental augmentation for retained exemplars. As demonstrated in the Example, the method has been validated on a real-world dataset of colorectal carcinoma histology images.
The present disclosure provides a computer-implemented method for improving classification performance of a neural network module adapted for learning over temporally spaced inputs of imaging data, said method comprising the steps of:
a) receiving an imaging data set, said set comprising data for a set of images that have a classification label assigned;
b) training a neural network module (100) on the data set of images from step (a), and storing the resulting neural network module;
c) applying Mobius data augmentation to one or more images from the data set of step (a) and storing the resulting transformed images;
d) receiving a new imaging data set, said set comprising data for a set of images that have a classification label assigned;
e) training the neural network module (100) on a combination of the imaging data obtained in step (c) and steps (d) and storying the resulting neural network module.

By neural network module is being understood the set of instructions describing the architecture of such neural network and the associated data files that contain information on the nodes of such neural network.

In one embodiment, the additional data set of images in step (d) comprises images of the same classes present in the original data set of step (a). This would allow to improve the classification accuracy of the neural network with respect of the same set of classes.

In one embodiment, to achieve continuous training of the neural network, the additional data set of images in step (d) comprises images of the classes present in the original data set of step (a) but also images belonging to new classes (incremental classes). In one embodiment, the new data set of images comprises at least one image of a new class not yet presented to the neural network.

The method is applied to medical imaging data. Specifically, as a proof of concept, the method has been applied to a set of histology images. Such method could be applied to 3D images as well, such as MRI or CT images. One approach to adapt the method to use 3D images is to transform those into 2D space by slicing them.

When the network has been already trained on a set of imaging data, the method involves steps (c-e) can be repeated with the subsequent new images received at later time point from step (d) being added to the augmented set of step (c). In such case the present disclosure a computer-implemented method for improving classification performance of a neural network module that has been pre-trained on a set of imaging data, said neural network module adapted for learning over temporally spaced inputs of imaging data, said method comprising the steps of:
a) applying Mobius data augmentation to one or more imaging data from a data set that have been already used to train a neural network module (100), said imaging data having a classification label assigned for each image, and storing the resulting transformed imaging data;
b) receiving a new imaging data set, said set comprising data for a set of images that have a classification label assigned.
c) updating the neural network module by training the neural network (100) on a combination of the imaging data obtained in step (a) and steps (b) and storying the resulting neural network module.

In one embodiment, the additional data set of images in step (b) comprises images of the same classes present in the original data set of step (a). This would allow to improve the classification accuracy of the neural network with respect of the same set of classes.

In one embodiment, to achieve continuous training of the neural network, the additional data set of images in step (b) comprises images of the classes present in the original data set used to generate imaging data of step (a) but also images belonging to new classes (incremental classes). In one embodiment, the new data set of images comprises at least one image of a new class not yet presented to the neural network.

Such methods could be implemented as a part of an image analysis system which is adapted for continuous learning and improvements of the classification performance at subsequent time points.

### Image analysis system

It will apparent that the method described above can be implemented on an image analysis system. For example, medical image analysis system. Such system must be able to receive images which have been classified and provide incremental training of the neural network using new images and previous images that have been processed by applying Mobius augmentation.

### Implementation of the method

It will be appreciated that the disclosure also applies to computer programs, particularly computer programs on or in a carrier, adapted to put the systems and the methods of the invention into practice. The present invention further provides a computer program comprising code means for performing the steps of the method described herein, wherein said computer program execution is carried on a computer. The present invention further provides a non-transitory computer-readable medium storing thereon executable instructions, that when executed by a computer, cause the computer to execute the method for incremental training of a deep learning model as described herein. The present invention further provides a computer program comprising code means for the elements of the system disclosed herein, wherein said computer program execution is carried on a computer.

The computer program may be in the form of a source code, an object code, a code intermediate source. The program can be in a partially compiled form, or in any other form suitable for use in the implementation of the method and it variations according to the invention. Such program may have many different architectural designs. A program code implementing the functionality of the method or the system according to the invention may be sub-divided into one or more sub-routines or sub-components. Many different ways of distributing the functionality among these sub-routines exist and will be known to the skilled person. The sub-routines may be stored together in one executable file to form a self-contained program. Alternatively, one or more or all of the sub-routines may be stored in at least one external library file and linked with a main program either statically or dynamically, e.g. at run-time. The main program contains at least one call to at least one of the sub-routines. The sub-routines may also call each other.

The present invention further provides a computer program product comprising computer-executable instructions implementing the steps of the methods set forth herein or its variations as set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files.

Computer readable media suitable for storing computer program instructions and data include all forms of nonvolatile memory, media and memory devices, including by way of example semiconductor memory devices (e.g., EPROM, EEPROM, and flash memory devices); magnetic disks; magneto optical disks; and CD ROM and DVD-ROM disks. The processor and the memory can be supplemented by, or incorporated in, special purpose logic circuitry.

It should be noted that the above-mentioned embodiments illustrate rather than limit the invention, and that those skilled in the art will be able to design many alternative embodiments without departing from the scope of the claims. In the claims, any reference signs placed between parentheses shall not be construed as limiting the claim. Use of the verb "comprise" and its conjugations does not exclude the presence of elements or steps other than those stated in a claim.

The article "a" or "an" preceding an element does not exclude the presence of a plurality of such elements. The invention may be implemented by means of hardware comprising several distinct elements, and by means of a suitably programmed computer. In the system claim enumerating several elements, several of these elements (sub-systems) may be embodied by one and the same item of hardware. The mere fact that certain measures are recited in mutually different dependent claims does not indicate that a combination of these measures cannot be used.

### Example implementation

Anonymized colorectal cancer HE stained tissue slides were obtained using an Aperio ScanScope scanner at 20x magnification. These are digitized and anonymized images of formalin-fixed paraffin embedded human colorectal adenocarcinomas and made publicly available through the pathology archives at the University Medical Center Mannheim [Kather, et al In Scientific reports, 2016]. These slides contain contiguous tissue areas that are manually annotated and tessellated. These are converted to 150 x 150 x 3 RGB patches. Overall, 5000 images were obtained for different tissue classes. In this study, 8 classes with 625 samples each were considered: 1. Tumor epithelium; 2. Simple stroma (homogeneous with tumor stroma, extra-tumoral stroma and smooth muscle); 3. Complex stroma (single tumor cells and immune cells); 4. Debris (necrosis, hemorrhage and mucus); 5. Immune cells (immune cell conglomerates and sub-mucosal lymphoid follicles); 6. Normal mucosal glands; 7. Adipose tissue; 8. Background. The base classes are the tumour epithelium (TE), simple stroma (SS), Immune cells (IC) and Adipose tissue (AT). The incrementally learnt classes include complex stroma (CS), debris (De), normal mucosal glands (NMG) and background (BG).

The experiment is split into two sequential tasks, labeled Task 1 and Task 2. The initial task proceeds with a standard cross-entropy objective and Task 2, the incremental task utilizes a joint loss with a cross-entropy term and a distillation loss. We utilize a ResNet-50 feature extractor, removing layers subsequent to the last residual block and adding to the last residual block a fully-connected (FC) layer of 512 units, followed by a FC layer with 4 units (number of classes) and loss heads. The pre-softmax layer generates probability scores by a sigmoid operation. An 80:20 split is used for the *train test* split on the dataset. Input images are resized to 224 x 224 and a batch size of 50 is used with a learning rate of 0.001 and adaptive moment optimization (Adam) [26]. Task 1 models are trained for 150 epochs on a (*N,label*) set for all *N* frames. In Task 2, models are trained for 150 epochs on (*N',label,logit*) tuples- *N'* having Mobius transformed versions of selectively retained old samples besides new class data. Note that we don't perform training time data augmentation except for the retained samples in incremental training. This is a departure from most machine learning efforts in clinical imaging but our aim is to analyze specific effects of Mobius transformations on incremental learning performance with distillation and otherwise. Thus, boosting base model accuracy is not aimed in the study. We set *T= 4.0* after grid search in *T* ∈ [1,5]. Two 32 GB Nvidia V100 GPUs, 512MB RAM used with ResNet 50 based models with ~24.8 million parameters, average training time of 102s per epoch in both tasks. Mobius augmentation modules and deep models are coded in Python 3.7.1 and Tensorflow 2.0 respectively.

**Table 1. Accuracy (%) for task 1/Stage 1 classes, after Task 1 is trained for, and after task 2 is incrementally added in Stage 2. The difference in accuracies on the validation set of Task 1 classes represents forgetting on them due to Task 2 addition**

| **Stage** | ***Stage 1*** | | | | | **Stage 2** | | | | | ***ΔAcc*** |
|---|---|---|---|---|---|---|---|---|---|---|---|
| | TE | SS | IC | AT | Avg(T1) | TE | SS | IC | AT | Avg(T2) | *T2-T1* |
| Our(MT+wKD) | 91.66 | 90.40 | 87.35 | 88.72 | 89.53 | 90.33 | 87.50 | 85.46 | 87.15 | 87.61 | 1.92 |
| Our(MT+KD) | 91.66 | 90.40 | 87.35 | 88.72 | 89.53 | 84.67 | 83.15 | 81.25 | 80.67 | 82.44 | 7.09 |
| Our (KD) | 91.66 | 90.40 | 87.35 | 88.72 | 89.53 | 77.20 | 72.33 | 70.54 | 73.25 | 73.33 | 16.20 |
| Our (MT+FT) | 91.66 | 90.40 | 87.35 | 88.72 | 89.53 | 75.24 | 68.10 | 67.11 | 70.77 | 70.31 | 19.22 |
| Ours (FT) | 91.66 | 90.40 | 87.35 | 88.72 | 89.53 | 55.45 | 48.67 | 47.05 | 51.10 | 50.57 | 38.96 |
| LwF.ewc [15] | 91.66 | 90.40 | 87.35 | 88.72 | 89.53 | 72.50 | 68.95 | 64.71 | 67.90 | 68.51 | 21.02 |
| LwM [11] | 91.66 | 90.40 | 87.35 | 88.72 | 89.53 | 76.95 | 73.85 | 69.20 | 73.35 | 73.34 | 16.19 |
| PDR [12] | 91.66 | 90.40 | 87.35 | 88.72 | 89.53 | 73.09 | 70.21 | 67.33 | 70.55 | 70.30 | 19.23 |

For the incremental task (Task 2), we use data from the 4 classes that are incrementally added. Mobius transformations for augmentation are exclusively applied to retained exemplars from Task 1 classes. Guided by memory constraints, we choose the top 20 examples for retention sorting by the magnitude of the class confidence scores after the validation set if passed through the trained models after Task 1. Trivially, including a greater number of samples can improve performance as theoretically shown in [2], with full joint training being an upper bound on incremental performance. Local storage conditions constrain our memory buffer available and we need to minimize memory use similar to several clinical imaging workflows worldwide. Thus, we stop at 20 instances for retention sets. The reduction in forgetting **(Table** 1) is pronounced for weighted distillation methods with a *ΔAcc* (difference in overall accuracy on Task 1 validation set before and after Task 2 training) of *1.92.* In Table 1, methods using both weighted distillation and Mobius augmentation are labeled as *'Our(MF+wKD)',* and as *'Our(MT+KD)'* if using unweighted distillation. *'Our(MT+FT)'* is the method where finetuning is combined with Mobius augmentation. For incremental tasks and in the overall accuracies for all classes after Task 2 training concludes, significant gains are seen with methods using Mobius operations to retained exemplars for initial task classes before interspersing with incremental class batches both for distillation and finetuning approaches. Overall, a clear advantage is seen when using distillation compared to finetuning alone. The best results are seen for combined distillation and Mobius aug-mentation before incremental optimization. This underscores the value of augmentation of old retained samples. This is different from most distillation-based methods that retain some old samples without incremental augmentation for retained samples while data augmentation is used only in initial sessions and the new incremental data.

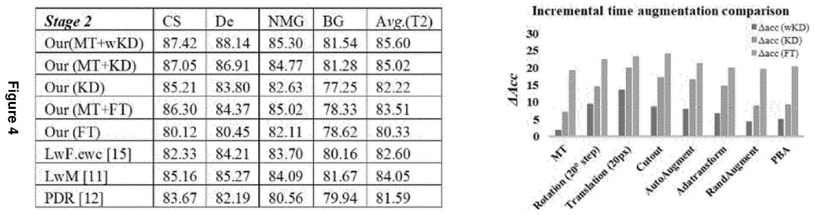

Baselines from literature are used with ResNet-50 backbones and original incremental training configurations molded to suit the two-task incremental aspect of our study. **Figure** 4 shows the performance of final model on Task 2. While one may convention-ally expect to have near equal accuracies across methods, we see slight differences in prediction accuracies within same Task 2 classes. The forward transfer effects of Task 1 training coupled with distillation based regularization is more optimal when using an intermediate Mobius augmentation step on old examples creating a diverse sample set for incremental training. Distilled models perform better on the new task overall due to distillation induced regularizations on parameter shifts unlike the unregularized optimization in finetuning (FT). We also compare (Table *2;right)* Mobius transformation based incremental augmentation (MT) with other augmentation ideas like cutout [DeVries, et al arXiv preprint arXiv:1708.04552, 2017], Adatransform [Tang, et al Proceedings of the IEEE International Conference on Computer Vision, pages 2998-3006, 2019], AutoAugment [Cubuk et al In Proceedings of the IEEE conference on computer vision and pattern recognition, pages 113-123, 2019], Population Based Augmentation (PBA) [Ho et al arXiv preprint arXiv:1905.05393, 2019], RandAugment [Cubuk et al arXiv preprint arXiv:1909.13719, 2019], rotation with 20° steps and translation with a 10px window. This comparison of *Δacc* values shows Mobius augmentation outperforming several sample-level methods in reducing forgetting by augmenting old task samples prior to incremental training. Future work can focus on studying the efficacy of Mobius transforms on other tasks like segmentation, comparing to generative augmentation methods and exploring Mobius augmentation in combination with concurrent methods in literature.

## Claims

1. A computer-implemented method for improving classification performance of a neural network module (100) that has been pre-trained on a data set of imaging data, wherein the data set of imaging data is a data set of medical images, said neural network module (100) adapted for learning over temporally spaced inputs of imaging data, said method comprising the steps of:
a) applying Mobius data augmentation to one or more images from the data set of imaging data that have been already used to pre-train the neural network module (100), said images having a classification label assigned for each image, and storing the resulting transformed images;
b) receiving a new imaging data set, said set comprising data for a set of images that have a classification label assigned; and
c) updating the neural network module (100) by training the neural network module (100) on a combination of the images obtained in step (a) and steps (b) and storing the resulting neural network module (100).

2. The method of claim 1, wherein steps (a-c) are repeated with the subsequent new images from step (b) being added to the augmented set of step (a).

3. The method of claim 1, wherein the additional data set of images in step (b) comprises images of the same classes as were already used to train the neural network module.

4. The method of claim 1, wherein the additional data set of images in step (b) comprises images of the classes present in the original data set used to train said neural network module (base classes) but also images belonging to new classes (incremental classes).

5. The method of claim 1, wherein the medical images are 2D medical images.

6. The method of claim 1, wherein the medical images are histology images.

7. The method of any preceding claim, wherein the one or more medical images from the data set of medical imaging data that have been already used to pre-train the neural network module (100) are selected based on a magnitude of a class confidence score for the medical images from the pre-trained neural network module (100).

8. The method of any preceding claim, wherein the training of the neural network module (100) in step (c) further uses validation logits from the pre-training of the neural network module (100).

9. The method of claim 8, wherein the validation logits are weighted using inverses of class-specific validation accuracies.

10. A image analysis system comprising:
an image acquisition device;
a machine-readable medium to store a neural network module (100);
a one or more processors that are configured to perform the steps of the method of claim 1.

11. The image analysis systems of claim 10, further comprising a user interface configured to allow user to classify images produced by the image acquisition device.

12. A machine-readable medium including instructions, which when executed by a processor, cause the processor to perform the steps of the method of claim 1.

## Patentansprüche

1. Computerimplementiertes Verfahren zur Verbesserung der Klassifizierungsleistung eines neuronalen Netzwerkmoduls (100), das anhand eines Datensatzes von Bilddaten vortrainiert wurde, wobei der Datensatz von Bilddaten ein Datensatz medizinischer Bilder ist, wobei das neuronale Netzwerkmodul (100) dafür ausgelegt ist, anhand zeitlich beabstandeter Eingaben von Bilddaten zu lernen, wobei das Verfahren folgende Schritte umfasst:
a) Anwenden einer Möbius-Datenaugmentierung auf ein oder mehrere Bilder aus dem Datensatz von Bilddaten, die bereits zum Vortrainieren des neuronalen Netzwerkmoduls (100) verwendet wurden, wobei den Bildern jeweils eine Klassifikationskennzeichnung zugewiesen ist, und Speichern der resultierenden transformierten Bilder;
b) Empfangen eines neuen Bilddatensatzes, wobei der Datensatz Daten für eine Reihe von Bildern umfasst, denen jeweils eine Klassifikationsbezeichnung zugewiesen ist; und
c) Aktualisieren des neuronalen Netzwerkmoduls (100) durch Trainieren des neuronalen Netzwerkmoduls (100) anhand einer Kombination der in Schritt (a) und Schritt (b) erhaltenen Bilder und Speichern des resultierenden neuronalen Netzwerkmoduls (100).

2. Verfahren nach Anspruch 1, wobei die Schritte (a-c) wiederholt werden, wobei die nachfolgenden neuen Bilder aus Schritt (b) dem erweiterten Satz aus Schritt (a) hinzugefügt werden.

3. Verfahren nach Anspruch 1, wobei der zusätzliche Bilddatensatz in Schritt (b) Bilder derselben Klassen umfasst, wie sie bereits zum Trainieren des neuronalen Netzwerkmoduls verwendet wurden.

4. Verfahren nach Anspruch 1, wobei der zusätzliche Bilddatensatz in Schritt (b) Bilder der Klassen, die im ursprünglichen, zum Trainieren des neuronalen Netzwerkmoduls verwendeten Datensatz vorhanden sind (Basisklassen), aber auch Bilder umfasst, die zu neuen Klassen gehören (inkrementelle Klassen).

5. Verfahren nach Anspruch 1, wobei es sich bei den medizinischen Bildern um 2D-medizinische Bilder handelt.

6. Verfahren nach Anspruch 1, wobei die medizinischen Bilder histologische Bilder sind.

7. Verfahren nach einem der vorhergehenden Ansprüche, wobei das eine oder die mehreren medizinischen Bilder aus dem Datensatz medizinischer Bilddaten, die bereits zum Vortrainieren des neuronalen Netzwerkmoduls (100) verwendet wurden, basierend auf der Größe eines Klassenkonfidenzwerts für die medizinischen Bilder aus dem vortrainierten neuronalen Netzwerkmodul (100) ausgewählt werden.

8. Verfahren nach einem der vorhergehenden Ansprüche, wobei das Trainieren des neuronalen Netzwerkmoduls (100) in Schritt (c) ferner Validierungs-Logits aus dem Vortraining des neuronalen Netzwerkmoduls (100) verwendet.

9. Verfahren nach Anspruch 8, wobei die Validierungs-Logits unter Verwendung der Kehrwerte klassenspezifischer Validierungsgenauigkeiten gewichtet werden.

10. Bildanalysesystem, das umfasst:
eine Bildaufnahmevorrichtung;
ein maschinenlesbares Medium zum Speichern eines neuronalen Netzwerkmoduls (100);
einen oder mehrere Prozessoren, die so konfiguriert sind, dass sie die Schritte des Verfahrens nach Anspruch 1 ausführen.

11. Bildanalysesystem nach Anspruch 10, das ferner eine Benutzerschnittstelle umfasst, die so konfiguriert ist, dass sie es einem Benutzer ermöglicht, von der Bildaufnahmevorrichtung erzeugte Bilder zu klassifizieren.

12. Maschinenlesbares Medium, das Anweisungen enthält, die, wenn sie von einem Prozessor ausgeführt werden, den Prozessor veranlassen, die Schritte des Verfahrens nach Anspruch 1 auszuführen.

## Revendications

1. Procédé mis en œuvre par ordinateur destiné à améliorer la performance de classification d'un module de réseau neuronal (100) ayant été pré-entraîné sur un ensemble de données d'imagerie, l'ensemble de données d'imagerie étant un ensemble de données d'images médicales, ledit module de réseau neuronal (100) étant adapté à un apprentissage à partir d'entrées de données d'imagerie espacées dans le temps, ledit procédé comprenant les étapes suivantes :
a) l'application de l'augmentation de données de type Möbius à une ou plusieurs images issues de l'ensemble de données d'imagerie ayant déjà été utilisées pour pré-entraîner le module de réseau neuronal (100), lesdites images présentant une étiquette de classification attribuée à chaque image, et le stockage des images transformées qui en résultent,
b) la réception d'un nouvel ensemble de données d'imagerie, ledit ensemble comprenant des données relatives à un ensemble d'images auxquelles est attribuée une étiquette de classification, et
c) l'actualisation du module de réseau neuronal (100) par entraînement du module de réseau neuronal (100) sur une combinaison desdites images obtenues aux étapes (a) et (b), et le stockage du module de réseau neuronal (100) qui en résulte.

2. Procédé selon la revendication 1, dans lequel les étapes (a) à (c) sont répétées avec les nouvelles images suivantes issues de l'étape (b) qui sont ajoutées à l'ensemble amélioré de l'étape (a).

3. Procédé selon la revendication 1, dans lequel l'ensemble de données d'images supplémentaire de l'étape (b) comprend des images des mêmes classes que celles qui ont déjà été utilisées pour entraîner le module de réseau neuronal.

4. Procédé selon la revendication 1, dans lequel l'ensemble de données d'images supplémentaire de l'étape (b) comprend des images des classes présentes dans l'ensemble de données d'origine utilisé pour entraîner ledit module de réseau neuronal (classes de base), mais également des images appartenant à de nouvelles classes (classes incrémentielles).

5. Procédé selon la revendication 1, dans lequel les images médicales sont des images médicales en 2D.

6. Procédé selon la revendication 1, dans lequel les images médicales sont des images histologiques.

7. Procédé selon l'une quelconque des revendications précédentes, dans lequel la ou les images médicales issues de l'ensemble de données d'imagerie médicale qui ont déjà été utilisées pour pré-entraîner le module de réseau neuronal (100) sont sélectionnées en fonction de l'importance d'un score de confiance de classe propre aux images médicales issues du module de réseau neuronal (100) pré-entraîné.

8. Procédé selon l'une quelconque des revendications précédentes, dans lequel l'entraînement du module de réseau neuronal (100) à l'étape (c) utilise en outre des logits de validation issus du pré-entraînement du module de réseau neuronal (100).

9. Procédé selon la revendication 8, dans lequel les logits de validation sont pondérés au moyen d'inverses des précisions de validation spécifiques aux classes.

10. Système d'analyse d'images comprenant :
un dispositif d'acquisition d'images,
un support lisible par machine destiné au stockage d'un module de réseau neuronal (100),
un ou plusieurs processeurs conçus pour réaliser les étapes du procédé selon la revendication 1.

11. Système d'analyse d'images selon la revendication 10, comprenant en outre une interface utilisateur conçue pour permettre à l'utilisateur de classer des images produites par le dispositif d'acquisition d'images.

12. Support lisible par machine comprenant des instructions qui, lorsqu'elles sont exécutées par un processeur, amènent le processeur à réaliser les étapes du procédé selon la revendication 1.
